(19) Europäisches Patentamt
European Patent Office
Office européen des brevets

(11) EP 1 163 230 B1

(12) # EUROPÄISCHE PATENTSCHRIFT

(45) Veröffentlichungstag und Bekanntmachung des Hinweises auf die Patenterteilung:
**10.09.2003 Patentblatt 2003/37**

(21) Anmeldenummer: **00907671.2**

(22) Anmeldetag: **08.03.2000**

(51) Int Cl.[7]: **C07D 251/24**, C07C 257/12

(86) Internationale Anmeldenummer:
**PCT/EP00/02021**

(87) Internationale Veröffentlichungsnummer:
**WO 00/053586 (14.09.2000 Gazette 2000/37)**

(54) **FORMAMIDIN-SULFAT UND -PHOSPHAT SALZE, DEREN HERSTELLUNG UND DEREN VERWENDUNG ZUR SYNTHESE VON 1,3,5-TRIAZIN**

FORMAMIDINE SULFATE AND PHOSPHATE SALTS, THEIR PRODUCTION AND THEIR USE IN THE SYNTHESIS OF 1,3,5-TRIAZINE

SELS DE SULFATE ET DE PHOSPHATE DE FORMAMIDINE, LEUR PREPARATION ET LEUR UTILISATION POUR LA SYNTHESE DE 1,3,5-TRIAZINE

(84) Benannte Vertragsstaaten:
**AT BE CH CY DE DK ES FI FR GB GR IE IT LI LU MC NL PT SE**
Benannte Erstreckungsstaaten:
**SI**

(30) Priorität: **08.03.1999 DE 19910093**

(43) Veröffentlichungstag der Anmeldung:
**19.12.2001 Patentblatt 2001/51**

(73) Patentinhaber: **Degussa AG**
**83308 Trostberg (DE)**

(72) Erfinder:
- **GÜTHNER, Thomas**
  **D-83308 Trostberg (DE)**
- **KRAMMER, Doris**
  **D-83376 Truchtlaching (DE)**
- **GRAML, Bernd**
  **D-84518 Garching (DE)**

(74) Vertreter: **Weiss, Wolfgang, Dipl.-Chem. Dr. et al**
**Weickmann & Weickmann**
**Patentanwälte**
**Kopernikusstrasse 9**
**81679 München (DE)**

(56) Entgegenhaltungen:
**EP-A- 0 370 391** **DE-A- 2 650 658**
**DE-A- 3 808 767** **DE-A- 4 001 160**

- **ODO K. ET AL.: "A new method for the preparation of formamidine" JOURNAL OF ORGANIC CHEMISTRY, US, AMERICAN CHEMICAL SOCIETY, EASTON, Bd. 22, Dezember 1957 (1957-12), Seite 1715 XP000886420 in der Anmeldung erwähnt**
- **SCHAEFER F.C. ET AL.: "Synthesis of the sym-triazine system. I. Trimerization and cotrimerization of amidines" JOURNAL OF THE AMERICAN CHEMICAL SOCIETY, US, AMERICAN CHEMICAL SOCIETY, EASTON, Bd. 81, Nr. 3, 20. März 1959 (1959-03-20), Seiten 1466-1470, XP000886449**
- **CHEMICAL ABSTRACTS, vol. 78, no. 16, 23. April 1973 (1973-04-23) Columbus, Ohio, US; abstract no. 100166n, AVDYUNIN V.I. ET AL.: "Effect of iron oxide on combustion rate of mixtures with different perchlorates" Seite 165; Spalte 1; XP002136569 & U. S. NAT. TECH. INFORM. SERV., AD REP. (XADRCH);1972; (NO. 750989,); 13 PP., Wright-Patterson Air Force Base;Foreign Technol. Div.; Ohio**
- **MAIER T. ET AL.: "Eine einfache Synthese für s-Triazin" SYNTHESIS, DE, GEORG THIEME VERLAG, STUTTGART,1979, Seite 690 XP000886417 in der Anmeldung erwähnt**
- **CHEMICAL ABSTRACTS, vol. 110, no. 5, 30. Januar 1989 (1989-01-30) Columbus, Ohio, US; abstract no. 38634n, IMUDA J. ET AL.: "Formamidine formate as intermediate for cimetidine and its analogs" Seite 507; Spalte 2; XP002136570 & JP 63 192747 A (MITSUI PETROCHEMICAL INDUSTRIES, LTD.;JAPAN) 10. August 1988 (1988-08-10)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 163 230 B1

Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft Formamidin-Salze, Verfahren zu ihrer Herstellung und deren Verwendung zur Herstellung von 1,3,5-Triazin.

1,3,5-Triazin ist ein, seit 1895 bekannter, seit 1953 strukturell gesicherter, sehr vielseitiger Synthesebaustein, der z. B. zur Synthese von Pyrimidinen, substituierten 1,3,5-Triazinen, 1,2,4-Triazolen und für Formylierungsreaktionen eingesetzt werden kann (vgl. Ch. Grundmann, Angew. Chem. 75 (1963), 393).

Der kommerzielle Einsatz von 1,3,5-Triazin für diese Synthesen wurde Obislang dadurch verhindert, daß 1,3,5-Triazin nur sehr umständlich synthetisiert werden kann und bei den meisten Verfahren in unreiner, für die Weiterverarbeitung ungeeigneter Form anfällt. 1,3,5-Triazin kann gemäß dem Stand der Technik z. B. auf folgende Weisen hergestellt werden:

1) Durch Überführung von wasserfreier Blausäure in das "Sesquihydrochlorid" ($(HCN)_6$•$(HCl)_9$) und Umsetzung mit einer organischen Base wie z.B. Chinolin (vgl. Ch. Grundmann, A. Kreuzberger, J. Am. Chem. Soc. 76 (1954) 5646). Problematisch hierbei ist die Handhabung wasserfreier Blausäure sowie die bereits im Labormaßstab sehr heftige, schwer zu kontrollierende Reaktion.

2) Durch Abspaltung von $H_2S$ aus Thioformamid (vgl. R. Willstätter, T.Wirth, Chem. Ber. 42 (1909),1918). Dieses Verfahren liefert nur geringe Ausbeuten von maximal 20 %, zudem ist Thioformamid nur sehr schwer zugänglich und der entstehende Schwefelwasserstoff bei der Handhabung und Entsorgung problematisch.

3) Durch Umsetzung von Formamidinacetat bzw. Ammoniumacetat mit Orthoameisensäureester (vgl. Th. Maier, H. Bredereck, W. Kantlehner, Synthesis 1979, 690 und DE 2 650 658 A). Dieses Verfahren erfordert einen hohen Anteil an Hilfsreagenzien, liefert gemäß der Beschreibung pro 100 kg Rohstoffe nur rund 10 kg Produkt, benötigt technisch schwierig zu erreichende Temperaturen von -70 °C und hat sich in der Praxis als nicht in der beschriebenen Weise nachvollziehbar erwiesen.

4) Durch thermische Zersetzung eines Formamidinsalzes zu 1,3,5-Triazin und dem korrespondierenden Ammoniumsalz nach folgender Gleichung (vgl. Ch. Grundmann, H. Schröder, W. Ruske, Chem. Ber. 87 (1954) 1865):

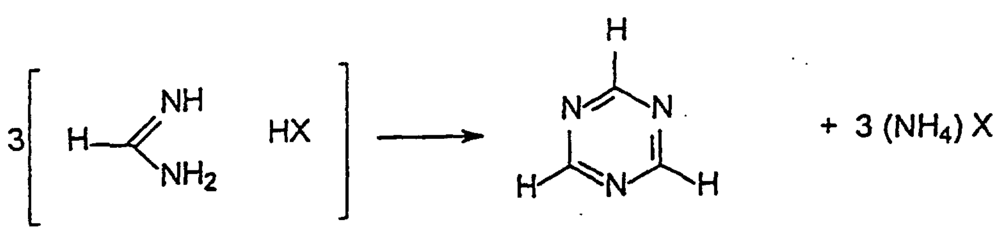

Dieses Verfahren zeichnet sich durch gut zugängliche Rohstoffe, einfache Synthesebedingungen sowie durch befriedigende Ausbeuten aus. Unglücklicherweise weisen jedoch die bekannten und technisch zugänglichen Formamidinsalze, das Acetat bzw. das Chlorid einen schwerwiegenden Nachteil auf. Das bei der thermischen Zersetzung mitentstehende Ammoniumsalz (Ammoniumacetat bzw. Ammoniumchlorid) besitzt einen relativ hohen Dampfdruck und sublimiert bereits merklich bei Temperaturen und Drücken, die zur Durchführung obigen Verfahrens notwendig sind. Dies führt dazu, daß bei der Zersetzung der entsprechenden Formamidin-Salze kein reines 1,3,5-Triazin isoliert werden kann, sondern ein Gemisch mit dem entsprechenden Ammoniumsalz erhalten wird.

Auch F.C. Schaefer et al. beschreiben in J. Am. Chem. Soc. 81 (1959), 1466-1470 die Synthese von *syn*-Triazin ausgehend von Formamidin-Hydrochlorid.

Die Herstellung von Formamidinacetat wird beispielsweise in DE 3 808 767 A1 durch katalytische Hydrierung von Cyanamid in wässriger, essigsauerer Lösung beschrieben. In Chemical Abstracts, 78, Nr. 16, 1973, Abstract Nr. 100166n, wird ein Formamidinperchlorat-Salz offenbart. In Chemical Abstracts, 110, Nr. 5, 1989, Abstract Nr. 38634n wird Formamidinformiat als Intermediat für Cimetidin und Analoga davon beschrieben. In der europäischen Patentanmeldung Nr. O 370 391 A2 werden Formamidinium-Salze zur Herstellung von 4,5-Dichlor-6-ethylpyrimidin verwendet. In keinem der Dokumente findet sich ein Hinweis auf ein verbessertes Verfahren zur Herstellung von 1,3,5-Triazin.

Geeignete Formamidinsalze, deren korrespondierende Ammoniumsalze eine ausreichend geringe Flüchtigkeit aufweisen, sind gemäß dem Stand der Technik nicht bekannt. Beispielsweise besitzen bekannte Formamidinsalze,

wie das Fluorid, Chlorid, Bromid, Formiat oder Acetat, eine ausgeprägte Sublimationsneigung. Bekannte Formamidinsalze mit oxidierenden Anionen, wie z.B. Perchlorat, Periodat, Nitrat, besitzen einen explosiven Charakter und scheiden daher für technische Anwendungen aus. Andere bekannte Formamidinsalze, wie z.B. das Benzolsulfonat, das 2-Hydroxyacetat, das Methyl-methylphosphonat oder das Methylsulfat sind aufgrund der hohen Kosten für das Anion, das letztlich als Ammoniumsalz entsorgt werden muß, technisch nicht geeignet.

Zusammenfassend ergibt sich, daß keines der gemäß dem Stand der Technik bekannten Verfahren geeignet ist, 1,3,5-Triazin kostengünstig in technisch durchführbarer Weise herzustellen.

Odo et al. (J. Org. Chem. 22 (1957), 17115) beschreiben ein Verfahren zur Herstellung von Formamidin-Sulfat oder Formamidin-Hydrochlorid durch katalytische Reduktion von Cyanamid in Gegenwart von $H_2SO_4$ oder HCl. Es findet sich kein Hinweis auf die Verwendung von Formamidin-Sulfat zur Herstellung von 1,3,5-Triazin.

Der vorliegenden Erfindung lag daher die Aufgabe zugrunde, neue Verfahren zur Herstellung von 1,3,5-Triazin zu entwickeln, welche die genannten Nachteile entsprechend dem Stand der Technik nicht aufweisen, sondern technisch einfach sind und die Gewinnung des Produkts in hoher Ausbeute und Reinheit ermöglichen.

Diese Aufgabe wurde erfindungsgemäß durch die Verwendung von Formamidin-Salzen entsprechend Anspruch 1 als Ausgangsmaterialien zur Herstellung von 1,3,5-Triazin gelöst.

Überraschenderweise wurde gefunden, daß die thermische Zersetzung von Formamidin-Salzen dann in technisch günstiger Weise durchführbar ist und zu hohen Ausbeuten von 1,3,5-Triazin führt, wenn als Formamidin-Salze Sulfate oder Phosphate eingesetzt werden.

Die zur Herstellung von 1,3,5-Triazin geeigneten Formamidin-Salze entsprechen der allgemeinen Formel I

$$(NH_2 - CH = NH_2)_n \; H_{x-n}Y \qquad (I)$$

wobei Y = $SO_4$, x = 2 sowie n = 0,1 bis 2,0
oder Y = $PO_4$, x = 3 sowie n = 0,1 bis 3,0
bedeuten.

Vorzugsweise stellen im Falle von Y = $SO_4$ n = 1 oder 2 oder im Falle von Y = $PO_4$ n = 1 oder 2 dar.

Ein weiterer Gegenstand der Erfindung sind neue Formamidin-Salze entsprechend der Formel Ia:

$$(NH_2\text{-}CH = NH_2)_nH_{x-n}Y \qquad (Ia)$$

wobei Y = $SO_4$, x = 2 sowie n = 1,0
oder Y = $PO_4$, x = 3 sowie n = 0,1 bis 3,0
bedeuten.

Die Herstellung der erfindungsgemäßen Formamidin-Salze kann auf verschiedenen Wegen erfolgen. Beispielsweise kann Formamidin (als freie Base) mit der entsprechenden Säure ($H_2SO_4$ bzw. $H_3PO_4$) in einem geeigneten Lösemittel umgesetzt werden. Ein bevorzugtes Verfahren zur Herstellung der erfindungsgemäßen Formamidin-Salze beruht auf der Umsetzung von Formamidin-Carboxylaten mit der entsprechenden Säure ($H_2SO_4$ bzw. $H_3PO_4$) in einem geeigneten Lösemittel bei Temperaturen von vorzugsweise 0 bis 50°C und besonders bevorzugt von 10 bis 30°C, wobei nach Abschluß der Umsetzung das Lösemittel sowie die freigesetzte Carbonsäure gemäß einer bevorzugten Ausführungsform abdestilliert werden. Besonders bevorzugt ist der Einsatz des großtechnisch verfügbaren Formamidin-Acetats.

Das stöchiometrische Verhältnis von Formamidin bzw. Formamidin-Carboxylat zu $H_2SO_4$ oder $H_3PO_4$ kann beliebig auf den jeweils gewünschten Wert eingestellt werden, so daß erfindungsgemäße Formamidinsalze innerhalb der gesamten für n angegebenen Bereiche erhältlich sind. Wenn n keine ganze Zahl ist, handelt es sich daher bei dem erfindungsgemäßen Formamidinsalz um eine Mischung von mindestens zwei Spezies mit jeweils unterschiedlicher Stöchiometrie, worin n eine ganze Zahl bedeutet.

Beispielhaft illustriert anhand der Umsetzung von Formamidin-Acetat mit Schwefelsäure zu Formamidin-Sulfat verläuft die Synthese nach folgender Reaktionsgleichung:

$$2\,(NH_2\text{-}CH{=}NH_2)\bullet OOC\text{-}CH_3 + H_2SO_4 \rightarrow (NH_2\text{-}CH{=}NH_2)_2\bullet SO_4 + 2\,HOOC\text{-}CH_3$$

Als Lösemittel für obige Umsetzung sind insbesondere Wasser, $C_1$- bis $C_5$-Alkohole, Ketone wie z. B. Aceton, oder $C_2$-$C_5$-Carbonsäuren oder Mischungen derselben geeignet. Bevorzugt sind Wasser bzw. die, dem eingesetzten Formamidin-Carboxylat entsprechende Carbonsäure. Besonders bevorzugt werden Wasser und Essigsäure.

Die auf diese Weise erhaltenen erfindungsgemäßen Formamidinsalze können vorteilhaft durch komplettes Verdampfen des eingesetzten Lösemittels sowie der freigesetzen Carbonsäure isoliert werden. Alternativ können sie durch partielles Verdampfen des Lösemittels, Kristallisation, Abtrennung und Trocknung des Salzes gewonnen werden.

Die erfindungsgemäßen Formamidinsalze können durch thermische Zersetzung zu 1,3,5-Triazin und dem entsprechenden Ammoniumsalz umgewandelt werden.

Die Zersetzungsreaktion kann bei einer Temperatur von 100 bis 200°C, vorzugsweise von 120 bis 170°C stattfinden. Die Zersetzungsreaktion kann bei Drücken zwischen 0,1 und 1 000 mbar erfolgen, bevorzugt sind Drücke von 5 bis 100 mbar.

Die Umsetzung kann unter Zusatz von inerten, als Wärmeüberträger dienenden Flüssigkeiten und/oder Feststoffen erfolgen. Beispiele für flüssige Wärmeüberträger sind Paraffine, Polysiloxane, aromatische Kohlenwassserstoffe oder Polyglykole. Beispiele für feste Wärmeüberträger sind Metalle, Glas, Keramik oder Graphit. Besonders bevorzugt ist jedoch die Ausführungsform, bei der kein zusätzlicher Wärmeüberträger verwendet wird.

Das bei der Reaktion entstehende 1,3,5-Triazin wird bevorzugt schnell aus dem Reaktionsbehälter durch Sublimation entfernt und durch Kondensation oder Resublimation in einer Vorlage abgeschieden. Die notwendige Temperatur zur Kondensation bzw. Resublimation hängt vom gewählten Druck ab und kann zwischen -80 und 114°C liegen. Um Produktverluste zu minimieren und unerwünschte Reaktionen zu vermeiden, wird eine Kondensations- bzw. Resublimationstemperatur von -10 bis -50°C bevorzugt.

Das bei der Kondensation bzw. Resublimation entstehende 1,3,5-Triazin besitzt eine hohe, für die meisten Anwendungen ausreichende Reinheit. Falls erforderlich kann es aus einem geeigneten Lösemittel umkristallisiert werden. Hierfür geeignete Lösemittel sind beispielsweise aliphatische und aromatische Kohlenwasserstoffe, aliphatische Ether und aliphatische Halogenkohlenwasserstoffe. Spezifische Beispiele für geeignete Lösemittel sind n-Pentan, n-Hexan, Cyclohexan, Benzol, Diethylether, t-Butylmethylether, Dichlormethan und Trichlormethan.

Die nachfolgenden Beispiele sollen dazu dienen, die Herstellung und Eigenschaften der erfindungsgemäßen Formamidinsalze zu erläutern sowie das Verfahren ihrer Verwendung zur Synthese von 1,3,5-Triazin zu illustrieren.

## Beispiel 1

### Herstellung von Formamidin-Hydrogensulfat

104 g (1 mol) Formamidinacetat wurden in 100 g Wasser gelöst. Bei 10°C wurden 140,1 g (1 mol) 70%ige Schwefelsäure zugetropft und die Lösung 30 Minuten nachgerührt. Die klare Lösung wurde bei 15 mbar Vakuum und 50 °C Badtemperatur einrotiert, der Rückstand im Vakuum bei 50 °C nachgetrocknet. Es wurden 142,1 g (100%) eines weißen, halbfesten Rückstands erhalten, der erst im Kühlschrank (4 °C) vollständig erstarrt.

Elementaranalyse: C 8,68, H 4,31, N 19,77 % (Theorie C 8,45, H 4,26, N 19,71 %). Der Formamidingehalt betrug 34 % (Theorie 33,3%).

## Beispiel 2

### Herstellung von Formamidin-Sulfat

208 g (2,0 mol) Formamidin-Acetat wurden in 208 g Wasser gelöst. Bei einer Temperatur von 10 °C wurden über 60 Minuten 140,1 g (1,0 mol) 70 %ige $H_2SO_4$ zugetropft und eine Stunde nachgerührt. Die klare Lösung wurde bei einem Vakuum von 20 mbar und einer Badtemperatur von 60°C am Rotationsverdampfer eingeengt. Als Rückstand wurden 186,4 g (100%) eines weißen, kristallinen Produkts erhalten.

Analysen: Gehalt Formamidin 47,8 % (Theorie: 47,32 %), Gehalt Sulfat 51,3 % (Theorie: 51,59 %). Der Schmelzpunkt betrug 160°C (unter Zersetzung). Elementaranalyse: C 12,94,H 5,54, N 29,96 % (Theorie: C 12,90, H 5,41, N 30,09 %)

## Beispiel 3

### Herstellung von Formamidin-Dihydrogenphosphat

104 g (1,0 mol) Formamidin-Acetat wurden in 104 g Wasser gelöst. Bei einer Temperatur von 10°C wurden über 60 Minuten 140 g (1,0 mol) 70 %ige $H_3PO_4$ zugetropft und eine Stunde nachgerührt. Die klare Lösung wurde bei einem Vakuum von 20 mbar und einer Badtemperatur von 60°C am Rotationsverdampfer eingeengt. Als Rückstand wurden 142,5 g (100 %) eines weißen, kristallinen Produkts erhalten.

Analysen: Gehalt Formamidin 31,1 % (Theorie: 31,02 %). Elementaranalyse: C 8,19, H 4,74, N 19,42 % (Theorie: C 8,46, H 4,97, N 19,72 %). Schmelzpunkt 43 bis 46°C.

**Beispiel 4**

**Herstellung von Di-formamidin-Monohydrogenphosphat**

208 g (2,0 mol) Formamidin-Acetat wurden in 104 g Wasser gelöst. Bei einer Temperatur von 10°C wurden über 60 Minuten 140g (1,0 mol) 70 %ige $H_3PO_4$ zugetropft und eine Stunde nachgerührt. Die klare Lösung wurde bei einem Vakuum von 15 mbar und einer Badtemperatur von 55°C am Rotationsverdampfer eingeengt und im Vakuum nachgetrocknet. Als Rückstand wurden 186,6 g (100%) eines weißen, kristallinen Produkts erhalten.

Analysen: Gehalt Formamidin 47,1 % (Theorie: 47,34 %).

Elementaranalyse:
C 12,75, H 5,98, N 29,37 % (Theorie: C 12,91, H 5,96, N 30,11 %). Schmelzpunkt 147 bis 150 °C

**Beispiel 5**

**Herstellung von 1,3,5-Triazin aus Formamidin-Sulfat**

93,0 g (0,5 mol) Formamidin-Sulfat aus Beispiel 2 wurden trocken, ohne weiteren Zusatz, in einem Dreihalskolben mit Rührer und isolierter Destiliationsbrücke unter einem Vakuum von 15 mbar auf 150 bis 160°C erhitzt. Innerhalb von 5 Stunden sammelten sich in der auf -20 °C gekühlten Vorlage 20,6 g weißes, kristallines 1,3,5-Triazin mit einer Reinheit von 98,2%. Die Ausbeute betrug 76 %. Im Reaktionsbehälter verblieben 70 g eines festen, bräunlichen Rückstands, der hauptsächlich aus Ammoniumsulfat bestand. 10 g des Produkts wurden aus t-Butylmethylether umkristallisiert. Es wurden 8,6 g 1,3,5-Triazin mit einer Reinheit von > 99,9 % erhalten.

**Beispiel 6 (Vergleich)**

**Herstellung von 1,3,5-Triazin aus Formamidin-Acetat**

104 g (1,0 mol) Formamidin-Acetat wurden in einem Dreihalskolben mit Rührer und isolierter Destillationsbrücke unter einem Vakuum von 100 mbar auf 130 bis 140 °C trocken erhitzt. Während der 3-stündigen Reaktion wurden in der auf -20 °C gekühlten Vorlage 76,1 g eines bräunlichen Sublimats erhalten. Im Reaktionskolben verblieben 24,5 g schwarzbrauner Rückstand. Das Sublimat bestand hauptsächlich aus Ammoniumacetat und enthielt nur 4,5 % 1,3,5-Triazin. Durch Extraktion mit n-Pentan und anschließendes Einengen wurden 2,7 g 1,3,5-Triazin als bräunliche Kristalle erhalten. Die isolierte Ausbeute betrug 10 % der Theorie.

**Beispiel 7 (Vergleich)**

**Herstellung von 1,3,5-Triazin aus Formamidin-Acetat und Triethylorthoformiat**

(gemäß Th. Maier, H. Bredereck, W. Kantlehner, Synthesis 1979, 690) 52 g (0,5 mol) Formamidin-Acetat und 148,2 g (1 mol) Triethylorthoformiat wurden in einer Destillationsapparatur während 5 Stunden auf 135 bis 140 °C erhitzt. Während dieser Zeit färbte sich der Inhalt des Reaktionskolbens schwarz und es wurden 107,0 g eines gelbbraunen Destillats erhalten. Dieses enthielt 3,2 % 1,3,5-Triazin. Durch Abkühlen auf -70 °C wurde daraus ein weißer Feststoff erhalten, der abgesaugt und mit n-Pentan gewaschen wurde. Nach dem Trocknen wurden 0,7 g eines feuchten, klebrigen Produkts erhalten, das einen 1,3,5-Triazin-Gehalt von 86 % aufwies. Ausbeute 2,2 % der Theorie.

**Patentansprüche**

1. Verwendung von Formamidin-Salzen der allgemeinen Formel (I)

$$(NH_2 - CH = NH_2)_n\ H_{x-n}Y \qquad (I)$$

wobei Y = $SO_4$, x = 2
sowie n = 0,1 bis 2,0
oder Y = $PO_4$, x = 3
sowie n = 0,1 bis 3,0

bedeuten,
zur Herstellung von 1,3,5-Triazin.

**2.** Verwendung nach Anspruch 1,
**dadurch gekennzeichnet,**
**daß** im Falle von $Y = SO_4$ n = 1 oder 2 darstellt.

**3.** Verwendung nach Anspruch 1,
**dadurch gekennzeichnet,**
**daß** im Falle von $Y = PO_4$ n für 1 oder 2 steht.

**4.** Verwendung nach einem der Ansprüche 1 bis 3,
**dadurch gekennzeichnet,**
**daß** die Formamidin-Salze bei Temperaturen von 100 bis 200°C und Drücken von 0,1 bis 1000 mbar thermisch zersetzt werden.

**5.** Verwendung nach einem der Ansprüche 1 bis 4,
**dadurch gekennzeichnet,**
**daß** man die thermische Zersetzung bei Temperaturen von 120 bis 170°C und Drücken von 5 bis 100 mbar durchführt.

**6.** Verwendung nach einem der Ansprüche 1 bis 5,
**dadurch gekennzeichnet,**
**daß** man das bei der thermischen Zersetzung entstehende 1,3,5-Triazin aus dem Reaktionsbehälter durch Sublimation entfernt und durch Kondensation in einer Vorlage abscheidet.

**7.** Verwendung nach Anspruch 6,
**dadurch gekennzeichnet,**
**daß** man die Kondensation bei Temperaturen von -10 bis -50°C vornimmt.

**8.** Verfahren zur Herstellung von Formamidin-Salzen der allgemeinen Formel (I)

$$(NH_2 - CH = NH_2)_n\ H_{x-n}Y \qquad (I)$$

wobei $Y = SO_4$, x = 2
sowie n = 0,1 bis 2,0
oder $Y = PO_4$, x = 3
sowie n = 0,1 bis 3,0
bedeuten,
**dadurch gekennzeichnet,**
**daß** man ein Formamidin-Carboxylat mit $H_2SO_4$ oder $H_3PO_4$ in Gegenwart eines Lösemittels bei Temperaturen von 0 bis 50°C, vorzugsweise von 10 bis 30°C umsetzt.

**9.** Verfahren nach Anspruch 8,
**dadurch gekennzeichnet,**
**daß** man als Formamidin-Carboxylat Formamidin-Acetat einsetzt.

**10.** Verfahren nach einem der Ansprüche 8 bis 9,
**dadurch gekennzeichnet,**
**daß** man als Lösemittel Wasser, $C_1$ bis $C_5$-Alkohole, Ketone, Carbonsäuren oder Mischungen davon verwendet.

**11.** Verfahren nach einem der Ansprüche 8 bis 10,
**dadurch gekennzeichnet,**
**daß** als Lösemittel ein Gemisch aus Wasser und Essigsäure eingesetzt wird.

**12.** Formamidin-Salze der allgemeinen Formel (Ia)

$$(NH_2\text{-}CH = NH_2)_n H_{x\text{-}n} Y \qquad (Ia)$$

wobei Y = $SO_4$, x = 2
sowie n = 1,0
oder Y = $PO_4$, x = 3
sowie n = 0,1 bis 3,0
bedeuten.

## Claims

**1.** Use of formamidine salts having the general formula (I)

$$(NH_2 - CH = NH_2)_n H_{x\text{-}n} Y \qquad (I)$$

in which Y = $SO_4$, x = 2
and n = 0.1 to 2.0
or Y = $PO_4$, x = 3
and n = 0.1 to 3.0
in the synthesis of 1,3,5-triazine.

**2.** Use according to claim 1,
**characterized in that**
if Y = $SO_4$, n = 1 or 2.

**3.** Use according to claim 1,
**characterized in that**
if Y = $PO_4$, n = 1 or 2.

**4.** Use according to one of the claims 1 to 3,
**characterized in that**
the formamidine salts are thermally decomposed at temperatures of 100 to 200 °C and pressures of 0.1 to 1000 mbar.

**5.** Use according to one of the claims 1 to 4,
**characterized in that**
thermal decomposition is carried out at temperatures of 120 to 170 °C and pressures of 5 to 100 mbar.

**6.** Use according to one of the claims 1 to 5,
**characterized in that**
the 1,3,5-triazine formed during the thermal decomposition is removed from the reaction vessel by means of sublimation and deposited by condensation in a receiver.

**7.** Use according to claim 6,
**characterized in that**
condensation is effected at temperatures from -10 to -50 °C.

**8.** Process for the production of formamidine salts having the general formula (I)

$$(NH_2 - CH = NH_2)_n H_{x\text{-}n} Y \qquad (I)$$

in which Y = $SO_4$, x = 2
and n= 0.1 to 2.0
or Y = $PO_4$, x = 3

and n= 0.1 to 3.0
**characterized in that**
a formamidine carboxylate is reacted with $H_2SO_4$ or $H_3PO_4$ in the presence of a solvent at temperatures from 0 to 50 °C, preferably from 10 to 30 °C.

**9.** Process according to claim 8,
**characterized in that**
formamidine acetate is used as formamidine carboxylate.

**10.** Process according to one of the claims 8 to 9,
**characterized in that**
water, $C_1$ to $C_5$ alcohols, ketones, carboxylic acids or mixtures thereof are used as solvent.

**11.** Process according to one of the claims 8 to 10,
**characterized in that**
a mixture of water and acetic acid is used as solvent.

**12.** Formamidine salts having the general formula (Ia)

$$(NH_2 - CH = NH_2)_n\ H_{x-n}Y \qquad \text{(Ia)}$$

in which Y = $SO_4$, x = 2
and n = 1.0
or Y = $PO_4$, x=3
and n = 0.1 to 3.0

## Revendications

**1.** Utilisation de sels de formamidine de formule générale (I)

$$(NH_2 - CH = NH_2)_n\ H_{x-n}Y \qquad \text{(I)}$$

où Y = $SO_4$, x = 2
et n = 0,1 à 2,0
ou bien Y = $PO_4$, x = 3
et n = 0,1 à 3,0,
pour la production de 1,3,5-triazine.

**2.** Utilisation selon la revendication 1 **caractérisée en ce que**, quand Y = $SO_4$, n représente 1 ou 2.

**3.** Utilisation selon la revendication 1 **caractérisée en ce que**, quand Y = $PO_4$, n représente 1 ou 2.

**4.** Utilisation selon l'une des revendications 1 à 3 **caractérisée en ce que** les sels de formamidine sont décomposés thermiquement à des températures de 100 à 200°C et à des pressions de 0,1 à 1000 mbar.

**5.** Utilisation selon l'une des revendications 1 à 4 **caractérisée en ce que** l'on réalise la décomposition thermique à des températures de 120 à 170°C et à des pressions de 5 à 100 mbar.

**6.** Utilisation selon l'une des revendications 1 à 5 **caractérisée en ce que** l'on retire par sublimation du récipient réactionnel la 1,3,5-triazine formée lors de la décomposition thermique et on la sépare par condensation dans un récipient récepteur.

**7.** Utilisation selon la revendication 6 **caractérisée en ce que** l'on réalise la condensation à des températures de -10 à -50°C.

**8.** Procédé de production de sels de formamidine de formule générale (I)

$$(NH_2 - CH = NH_2)_n\ H_{x-n}Y \qquad (I)$$

où Y = $SO_4$, x = 2
et n = 0,1 à 2,0
ou bien Y = $PO_4$, x = 3
et n = 0,1 à 3,0,
**caractérisé en ce que** l'on fait réagir un carboxylate de formamidine avec $H_2SO_4$ ou $H_3PO_4$ en présence d'un solvant à des températures de 0 à 50°C, de préférence de 10 à 30°C.

**9.** Procédé selon la revendication 8 **caractérisé en ce que** l'on utilise l'acétate de formamidine comme carboxylate de formamidine.

**10.** Procédé selon l'une des revendications 8 à 9 **caractérisé en ce que** l'on utilise comme solvant l'eau, des alcools en $C_1$ à $C_5$, des cétones, des acides carboxyliques ou des mélanges de ceux-ci.

**11.** Procédé selon l'une des revendications 8 à 10 **caractérisé en ce que** l'on utilise un mélange d'eau et d'acide acétique comme solvant.

**12.** Sels de formamidine de formule générale (Ia)

$$(NH_2 - CH = NH_2)_n\ H_{x-n}Y \qquad (Ia)$$

où Y = $SO_4$, x = 2
et n = 1,0
ou bien Y = $PO_4$, x = 3
et n = 0,1 à 3,0.